Europäisches Patentamt

European Patent Office

Office européen des brevets

(11) Veröffentlichungsnummer: **0 283 434**
**A2**

(12) # EUROPÄISCHE PATENTANMELDUNG

(21) Anmeldenummer: 88810107.8

(22) Anmeldetag: 22.02.88

(51) Int. Cl.⁴: **A 61 L 15/06**
**A 61 L 15/03**

(30) Priorität: 27.02.87 CH 755/87

(43) Veröffentlichungstag der Anmeldung:
21.09.88 Patentblatt 88/38

(84) Benannte Vertragsstaaten:
AT BE CH DE ES FR GB GR IT LI LU NL SE

(71) Anmelder: **CIBA-GEIGY AG**
**Klybeckstrasse 141**
**CH-4002 Basel (CH)**

(72) Erfinder: **Fankhauser, Peter, Dr.**
**Hauptstrasse 65**
**CH-4107 Ettingen (CH)**

**Sinnreich, Joel, Dr.**
**Hohe Winde Strasse 98**
**CH-4059 Basel (CH)**

**Dobmeier, Rolf**
**Drosselstrasse 25**
**CH-4103 Bottmingen (CH)**

Patentansprüche für folgende Vertragsstaaten: ES + GR.

(54) **Pharmazeutisches Pflaster.**

(57) Die Erfindung betrifft ein pharmazeutisches, auf der Schleimhaut haftendes Pflaster bestehend aus zwei diskreten, gut zusammenhaftenden Schichten, das pharmazeutische Wirkstoffe, die zur topischen Applikation oder zur systemischen Therapie geeignet sind, enthalten kann. Es zeichnet sich durch eine hervorragende, langdauernde Haftung auf der Schleimhaut und sehr angenehme Trageeigenschaften aus, insbesondere im Mund als Bukalpflaster.

EP 0 283 434 A2

**Beschreibung**

## Pharmazeutisches Pflaster

Die Verabreichung pharmazeutischer Wirkstoffe über die Schleimhaut (Mucosa), insbesondere die Munschleimhaut, hat in den letzten Jahren zunehmendes Interesse gefunden. Ein Hauptvorteil dieser Darreichungsform liegt im raschen Wirkungseintritt. Sie findet daher z.B. bei Angina pectoris Anwendung, wo es auf eine schnelle Anfallkupierung ankommt. Weiterhin ist es möglich, mit dieser Methode Wirkstoffe unter weitgehender Umgehung des Magen-Darm-Traktes zu applizieren. Sie ist daher in vielen Fällen eine Alternative zur parenteralen Verabreichung von Wirkstoffen: sie ist angenehmer als letztere und kann vom Patienten selbst angewendet werden.

Es sind verschiedene Möglichkeiten, eine Wirkstoff über die Schleimhaut zu applizieren, vorgeschlagen worden. Z.B. beschreibt EP-A-107.941 ein pharmazeutisches Präparat für den Mundraum, bei dem der Wirkstoff in eine weiche, homogene Masse eingebettet ist. Diese Masse besteht aus (a) einem wasserlöslichen Protein, das die Wirkstoffabsorption fördert, (b) einem Polyalkohol, (c), einem Fettsäureester oder/und (d) einem Carboxyvinyl-Polymer.

Das US-Patent Nr. 4.517.173 beschreibt ein aus mindestens zwei Schichten bestehendes Filmpräparat, das auf Schleimhäuten haftet. Es besteht im wesentlichen aus einer wasserlöslichen "pharmazeutischen Schicht", die wasserlösliche Hochpolymere, z.B. wasserlösliche Cellulosederivate, Polyacrylsäure-Copolymere, Polyvinylalkohol, Polyvinylpyrrolidon, Polyalkylenglykol, Hydroxypropylstärke, Alginsäure oder Salze davon, Polysaccharide oder Tragacanth, Gelatinegummi, denaturierte Gelatine oder Collagen, sowie pharmazeutische Wirkstoffe enthält und die auf der Schleimhaut haftet. Die äussere zweite Schicht zeigt einige Resistenz gegen Wasser. Sie besteht aus den obengenannten wasserlöslichen Hochpolymeren und wenig wasserlöslichen Komponenten, wie z.B. Schellack, höheren Fettsäuren oder schlecht wasserlöslichen Cellulosederivaten.

DE-A-3.237.945 beschreibt eine pharmazeutische Zusammensetzung mit verzögerter Freigabe im Mundbereich, die aus einer auf der Mundschleimhaut haftenden Schicht und einer nichthaftenden wasserlöslichen oder in Wasser zersetzbaren äusseren Schicht besteht, wobei der Wirkstoff in mindestens einer dieser beiden Schichten eingelagert ist. Die haftende Schicht enthält ein Haftmittel, z.B. wasserlöschlichen Celluolosegummi, etwa Natriumcarboxymethylcellulose. Die nichthaftende Schicht besteht z.B. aus Polyacrylsäure oder aus Copolymeren der Acrylsäure, hydrophilen Vinylpolymeren oder Polysacchariden, insbesondere Cellulosederivaten, z.B. Hydroxypropylcellulose.

JP-A-85-116630 beschreibt ein aus zwei Schichten bestehendes pharmazeutisches Präparat, das auf der Mundschleimhaut haftet. Die "medizinische Schicht" besteht aus (a) Polyacrylsäure oder Copolymeren der Acrylsäure, (b) Natriumcarboxymethylcellulose, Natriumalginat und/oder Hydroxyethylcellulose, (c) Glycerin und/oder Propylenglykol, und (d) dem Wirkstoff. Die zweite Schicht enthält die Bestandteile (a), (b) und (c) der "medizinischen Schicht", ferner anstelle des Wirkstoffs ein mindestens bivalentes pharmazeutisch tolerierbares Metallsalz.

EP-A-75540 beschreibt ein membranbildendes Assoziationspolymer, bestehend aus einer polymeren Carbonsäure, die an wenigstens 10% der Monomer-Einheiten freie Carboxygruppen trägt, und einem ethoxylierten, nichtionischen Tensid, das wenigstens 2 Ethylenoxid-Einheiten enthält, und ein Molekulargewicht von maximal 4000 besitzt. Dieses wird zur verzögerten Abgabe von systemisch wirksamen Pharmazeutika über die Haut oder Schleimhaut vorgeschlagen.

Demgegenüber betrifft die vorliegende Erfindung ein pharmazeutsches, auf der Schleimhaut haftendes Pflaster bestehend aus zwei diskreten, gut zusammenhaftenden Schichten:

(a) einer Abdeckfolie enthaltend als wesentliche Bestandteile:

(1) kaltwasserunlöslichen Polyvinylalkohol, in dem höchstens 10% der Hydroxygruppen als Acetatgruppen vorliegen; und

(2) gegebenenfalls einen oder mehrere Weichmacher; und

(b) einer Klebeschicht enthaltend als wesentliche Bestandteile:

(1) ein bei Körpertemperatur wasserunlösliches Assoziationspolymer, gebildet ($\alpha$) aus einer polymeren Carbonsäure, worin jeweils wenigstens 10 % der Monomereinheiten freie Carboxygruppen tragen, und ($\beta$) einem ethoxylierten, nichtionischen Tensid, das wenigstens 2 Ethylenoxid-Einheiten enthält und ein Molekulargewicht von maximal 4000 besitzt, in einem Gewichtsverhältnis von etwa 50:1 bis etwa 1:20;

gegebenenfalls einen oder mehrere pharmazeutische Wirkstoffe; und

(3) gegebenenfalls einen oder mehrere die Absorption des (der) pharmazeutischen Wirkstoffe(s) fördernde Substanzen;

Als Scheleimhäute kommen alle Mucosae des menschlichen oder tierischen Körpers in Frage, z.B. die Schleimhäute des Mundraums, der Nase, der Vagina, des Rectums oder Uterus. In erster Linie dient das erfundungsgemässe Pflaster als Bukalpflaster.

Polyvinylalkohol, in dem höchstens 10% der Hydroxygruppen als Acetatgruppen vorliegen, wird auch als Polyvinylalkohol mit einem Hydrolysegrad von mindestens 90% bezeichnet. Der Hydrolysegrad von Polyvinylalkohol bezieht sich auf die übliche Herstellung von Polyvinylalkohol durch Hydrolyse von Polyvinylacetat. Der nichthydrolysierte Anteil des erfindungsgemäss verwendeten Polyvinylalkohols besteht daher aus Polyvinylacetat-Monomereinheiten, d.h. 1-Acetoxy-1,2-ethylengruppen. Vorzugsweise liegen im verwendeten Polyvinylalkohol höchstens 8 % (Hydrolysegrad mindestens 92 %), insbesondere höchstens 5 % (Hydrolysegrad mindestens 95 %) und vor allem höchstens 2 % (Hydrolysegrad mindestens 98 %) der Hydroxygruppen als Acetatgruppen vor.

Assoziationspolymere, wie sie in der Klebeschicht Anwendung finden, sind z.B. in EP-A-75540 beschrieben. Der Inhalt dieser Patentanmeldung ist per Referenz mit eingeschlossen. Das Geweichtsverhältnis polymere Carbonsäure/ethyoxyliertes Tensid ist vorzugsweise 50:1 bis 1:10, insbesondere 20:1 bis 1:4 und vor allem 20:1 bis 5:1.

Der Begriff "polymere Carbonsäure" umfasst saure Polymermaterialien, in denen die Azidität auf freie Carboxygruppen zurückzuführen ist, und worin wenigstens 10 %, vorzugsweise wenigstens 30 % und in erster Linie wenigstens 50 %, und höchstens 100 % der Monomereinheiten freie Carboxygruppen tragen. Bevorzugte Vertreter sind Polyacrylsäure, Polymaleinsäure, Polyacrylsäure-Copolymere, Polymaleinsäure-Copolymere und Alginsäure.

Polyacrylsäure-Copolymere und Polymaleinsäure-Copolymere umfassen Copolymere der Acryl- bzw. Maleinsäure mit einer oder mehreren polymerisierbaren Vinyl- und/oder Vinylidenverbindungen, z.B. Vinylhalogenide, etwa Vinylchlorid- oder -bromid; Vinylester, z.B. Vinylacetat oder Vinylbenzoat; Acrylnitril; Vinylphenyl-Verbindungen, z.B. Styrol oder Vinyltoluol; Niederalkylester von Niederalkensäuren, z.B. Methylmethacrylat oder Ethylacrylat; Vinylketo-Verbindungen, z.B. Vinylmethylketon oder Methacrolein; Vinylether, z.B. Vinylmethylether; oder Amide, Niederalkylamide oder Diniederalkylamide von Niederalkensäuren, z.B. tert.-Butylacrylamid oder N,N-Dimethylacrylamid.

Die ethoxylierten, nichtionischen Tenside enthalten mindestens 2, vorzugsweise mindestens 5, vorzugsweise höchstens 30 und insbesondere höchstens 23 Ethylenoxideinheiten und besitzen HLB-Werte zwischen 2 und 40, vorzugsweise zwischen 4 und 18. Sie besitzen vorzugsweise ein durchschnittliches Molekulargewicht zwischen 150 und 4000, in erster Linie zwischen 150 und 3500. Bevorzugte Vertreter sind Polyoxyethylenfettalkohole und Polyoxyethylensorbitanfettsäuren.

Polyoxyethylenfettalkohole sind vorzugsweise polyoxyethylierte $C_8$-$C_{32}$-Fettalkohole, insbesondere $C_8$-$C_{24}$-Fettalkohole, z.B. ethoxylierter Stearyl- oder Palmitylalkohol.

Polyoxyethylensorbitanfettsäuren enthalten als Fettsäuren vorzugsweise solche ohne oder mit einer, insbesondere ohne, Doppelbindung und ferner vorzugsweise solche mit 8-32, insbesondere 8-24, Kohlenstoffatomen und umfassen z.B. ethoxyliertes Sorbitan-monolaurat, -monopalmitat, -monostearat, -monooleat, -tristearat oder -trioleat.

Falls gewünscht, kann die Abdeckfolie des erfindungsgemässen Pflasters galenisch akzeptable Weichmacher, beispielsweise hydrophile Weichmacher, in Konzentrationen von etwa 1-30 Gew.%, vorzugsweise 5-20 und insbesondere 10 Gew.-%, enthalten, um der Folie eine entsprechende Flexibilität zu geben. Hydrophile Weichmacher sind z.B. aliphatische Polyole, z.B. Glycerin, 1,3-Butandiol, 1,4-Butandiol, Propandiol, Ethylenglykol und Polyethylenglykol, vor allem Glycerin, oder auch Wasser. Falls gewünscht, kann auch die Klebeschicht einen oder mehrere der obengenannten hydrophilen Weichmacher enthalen.

Die Klebeschicht - und auch die Abdeckfolie - kann ferner andere pharmazeutische Additive, z.B. Farbpigmente, Bindemittel, aromatisierende Substanzen, Geschmacksstoffe, Süssmittel, andere Mittel zur Ueberdeckung eines unerwünschten Geschmacks herrührend vom Wirkstoff, Konservierungsmittel usw., enthalten.

Werden Farbpigmente angewendet, so kann man mit ihnen vorteilhaft die beiden Schichten des Pflasters - die nichthaftende Abdeckfolie und die Klebeschicht - kennzeichnen, z.B. dadurch dass die Abdeckfolie anders gefärbt ist als die Klebeschicht. Auf diese Weise wird die Anwendung des Pflasters erleichtert.

Mit Hilfe des erfindungsgemässen Pflasters können einzelne pharmazeutische Wirkstoffe und Kombinationen von Wirkstoffen verabreicht werden. Die Wirkstoffe werden bevorzugt in der Form verwendet, in der sie in der Schleimhaut, insbesondere Mundschleimhaut, genügend stabil sind. Für das erfindungsgemässe Pflaster befindet sich der Wirkstoff vorzugsweise in einer Form, die geschmacklos oder hinsichtlich des Geschmackes annehmbar ist.

Die zum Einsatz gelangenden pharmazeutischen Wirkstoffe können einmal zur topischen Applikation geeignet sein, zum anderen aber auch zur systemischen Therapie, wobei die Resorption des Wirkstoffes vor allem direkt durch die betreffende Schleimhaut, ferner generell im Mundbereich oder zu einem geringem Teil auch im Gastrointestinaltrakt, falls Wirkstoff verschluckt wird, erfolgt.

Die systemische Therapie mit dem erfindungsgemässen Pflaster ist insbesondere dann angezeigt, wenn ein Wirkstoff oral nicht zufriedenstellend verabreicht werden kann.

Bei der Auswahl und Dosierung des Wirkstoffes muss sichergestellt sein, dass ein versehentlich verschlucktes Pflaster nicht zu unbeabsichtigten Ueberdosierungen führt.

Beispiele für Wirkstoffe, die zur topischen Applikation geeignet sind, sind Analgetika, z.B. Acetylsalicylsäure, Antiinflammatorika, z.B. Diclofenac oder Salze davon, Lokalanästhetika, z.B. Lidocain, Novocain oder Nupercain, oder antivirale Mittel, z.B. ein Gemisch aus Heparin und Zinksalzen, insbesondere Heparin-natriumsalz und Zinksulfat-monohydrat, oder Acyclovir.

Beispiele für Wirkstoffe, die zur systemischen Therapie geeignet sind, sind Anästhetika, z.B. Morphin, Fentanyl und Derivate davon, z.B. Sufentanyl, Hormone, z.B. Progesteron, antianginöse Präparate, z.B. Nitroglycerin oder Isosorbid-dinitrat, Antiinflammatorika, z.B. Acetylsalicylsäure oder Diclofenac oder Salze davon, β-Blocker, z.B. Propranolol, Oxprenolol oder Metoprolol, Entwöhnungsmittel, z.B. Nikotin zur Raucherentwöhnung, Mittel gegen die Reisekrankheit, z.B. Scopolamin, oder Peptidwirkstoffe, z.B. Calcitonin, Vasopressin oder Oxytocin.

Die systemische Therapie kann gegebenenfalls durch den Zusatz von penetrationsfördernden, d.h. die Absorption der Wirkstoffe fördernden, Substanzen, z.B. Trypsin, Hyaluronidase, Natriumsalicylat, Natriumglykocholat oder n-Decylmethylsulfoxid, zur Klebeschicht unterstützt werden. Falls solche Substanzen in das Pflaster eingearbeitet werden, beträgt ihr Gewichtsanteil bevorzugt bis zu 60 %, insbesondere bis zu 20 % und vor allem 5 bis 20 % des Gesamtsystems.

Es ist möglich, dass sowohl die pharmazeutischen Wirkstoffe als auch die gegebenenfalls verwendeten penetrationsfördernden Substanzen, die ursprünglich nur in die Klebeschicht eingearbeitet werden, zum Teil auch in die benachbarte Abdeckfolie übergehen und sich ein Gleichgewicht ausbildet. Dieser Vorgang kann z.B. bei der Lagerung der fertiggestellten pharmazeutischen Pflaster ablaufen und beeinträchtigt die Funktion der Pflaster in keiner Weise.

Das Pflaster kann ferner, wenn es keinen pharmazeutischen Wirkstoff enthält, zur zuverlässigen und langdauernden Abdeckung von Läsionen, z.B. Schnitten, Abschürfungen oder Aphthen, der Schleimhaut dienen.

Die Dicke des erfindungsgemässen pharmazeutischen Pflasters ist nicht kristisch. Es ist bevorzugt, eine Abdeckfolie mit einer Dicke von ungefähr 0,03 bis 2 mm zu verwenden, insbesondere von 0,05 bis 0,2 mm, und eine Klebeschicht von ungefähr 0,02 bis ungefähr 1 mm Dicke, insbesondere von ungefähr 0,05 bis 0,3 mm Dicke. Das erfindungsgemässe Pflaster kann von jeder gewünschten Form und Dimension sein.

Die oben beschriebenen bekannten Präparate sind alle dadurch gekennzeichnet, dass sie wasserlösliche Elemente - Schichten, Bestandteile - enthalten. Das führt dazu, dass mit dem Zeitpunkt der Applikation im Mund der Auflösungsprozess des Präparates einsetzt. Eine frühe Abbröckelung oder vollständige Auflösung oder Ablösung von der Mundschleimhaut und Verschlucken des Präparates sind die Folgen. Demgegenüber zeichnet sich das erfindungsgemässe Pflaster dadurch aus, dass beide Schichten bei Körpertemperatur unlöslich sind. Dadurch wird erreicht, dass dieses Pflaster länger und zuverlässiger als jedes andere bekannte Präparat auf der Schleimhaut, insbesondere der Mundschleimhaut haftet.

Der in der Abdeckfolie verwendete kaltwasserunlösliche Polyvinylalkohol ist wasserquellbar. Im nassen, gequollenen Zustand ist er weich und flexibel und weist eine glatte, insbesondere für die Applikation im Mund angenehme Oberfläche auf. Da er nicht auf der Schleimhaut haftet, kann es nicht zu einem unerwünschten Festlkeben der "falschen" Seite des Pflasters im Mundraum kommen.

Das in der Klebeschicht zur Anwendung kommende Assoziationspolymer ist ebenfalls so wasserquellfähig, dass es mit fortschreitender Aufenthaltsdauer im Mundraum eine Masse von genügend geringer Kohärenz bildet, die es dem Patienten ermöglicht, das Pflaster schmerzfrei wieder abzulösen. Es ist jedoch auch ohne weiteres möglich, das Pflaster sofort nach der Fixierung im Mundraum wieder zu entfernen - etwa, weil es an einer ungeeigneten Stelle plaziert worden ist -, wobei dann nur etwas grössere Haftkräfte zu überwinden sind. Im letzteren Fall kann man dasselbe Pflaster nochmals verwenden und an einer geeigneten Stelle befesigen, da die Klebeschicht nur wenig oder noch gar nicht gequollen ist. Verzichtet der Patient ganz darauf, das Pflaster abzunehmen, so wird es schliesslich abfallen, und der Patient kann es entweder dem Mundraum entnehmen oder verschlucken.

Im Zuge der vorliegenden Erfindung wurde überraschend gefunden, dass kaltwasserunlöslicher Polyvinylalkohol sehr gut auf dem in der Klebeschicht verwendeten Assoziationspolymer haftet. Daher kann beim erfindungsgemässen Pflaster erstmals die hervorragende Eignung des kaltwasserunlöslichen Polyvinylalkohols als Abdeckschicht ausgenützt werden. Folien aus diesem Material quellen gerade im notwendigen Ausmass, so dass beim Darüberfahren mit der Zunge ein Gefühl der Weichheit und Glätte entsteht. Dadurch wird eine so weitgehende Anpassung des Bukalpflasters auf der Schleimhaut erreicht, dass der Patient das Pflaster nicht als "Fremdkörper" empfindet und sogar dessen Anwesenheit im Mundraum völlig aus dem Bewusstsein verdrängen, also "vergessen" kann.

Die Erfindung betrifft bevorzugt ein Pflaster bestehend aus:

(a) einer Abdeckfolie enthaltend als wesentliche Bestandteile:

kaltwasserunlöslichen Polyvinylalkohol, in dem höchstens 8 % der Hydroxygruppen als Acetatgruppen vorliegen; und

(2) 1-30 Gew.-% eines oder mehrerer Weichmacher; und

(b) einer Klebeschicht enthaltend als wesentliche Bestandteile:

(1) ein Assoziationspolymer, gebildet (α) aus einer polymeren Carbonsäure aus der Gruppe Polyacrylsäure und Alginsäure und (β) einem ethoxylierten, nichtionischen Tensid aus der Gruppe Polyoxyethylenfettalkohole und Polyoxyethylensorbitanfettsäuren, in einem Gewichtsverhältnis von etwa 20:1 bis etwa 1:4;

(2) gegebenenfalls einen oder mehrere pharmazeutische Wirkstoffe; und

(3) gegebenenfalls einen oder mehrere die Absorption des (der) pharmazeutischen Wirkstoffe(s) fördernde Substanzen.

Die Erfindung betrifft in erster Linie ein Pflaster bestehend aus:

(a) einer Abdeckfolie enthaltend als wesentliche Bestandteile:

(1) kaltwasserunlöslichen Polyvinylalkohol, in dem höchstens 2 % der Hydroxygruppen als Acetatgruppen vorliegen; und

4

(2) 5-20 Gew.-% eines oder mehrerer Weichmacher; und

(b) einer Klebeschicht enthaltend als wesentliche Bestandteile:

(1) ein Assoziationspolymer, gebildet ($\alpha$) aus Polyacrylsäure und ($\beta$) einem ethoxylierten, nichtionischen Tensid aus der Gruppe polyoxyethylierter Stearylalkohol, polyoxyethylierter Palmityl alkohol, Polyoxyethylensorbitanstearinsäure und Polyoxyethylensorbitanpalmitinsäure, in einem Gewichtsverhältnis von etwa 15:1 bis etwa 5:1;

(2) gegebenenfalls einen oder mehrere pharmazeutische Wirkstoffe; und

(3) gegebenenfalls einen oder mehrere die Absorption des (der) pharmazeutischen Wirkstoffe(s) fördernde Substanzen.

Das erfindungsgemässe Pflaster wird in an sich bekannter Weise hergestellt, z.B. dadurch, dass man die Abdeckfolie mit einer Klebemasse bestehend aus den Bestandteilen der Klebeschicht und einem Lösungsmittel gelichmässig beschichtet, das Lösungsmittel verdampft und das Produkt in die gewünschte Form bringt.

Das erfindungsgemässe pharmazeutische Pflaster kann in an sich bekannter Weise in einem kontinuierlichen oder einem entsprechenden nicht-kontinuierlichen Verfahren hergestellt werden.

In einem einfachen, nicht-kontinuierlichen Verfahren können die Bestandteile der Abdeckfolie in Wasser gelöst werden, wobei eine hochviskose Flüssigkeit erhalten wird, die danach auf eine glatte Oberfläche, beispielsweise eine Glas- oder Stahlplatte, aufgetragen wird. Nach dem Abdampfen des Wassers entsteht eine feste Folie. Die Bestandteile der Klebeschicht können in einem geeigneten Lösungsmittel, beispielsweise Ethanol, auf die Oberfläche der Abdeckfolie aufgetragen und das Lösungsmittel danach verdampft werden. Der letztere Vorgang kann auch mehrfach wiederholt werden. Das Produkt wird in die für die Applikation gewünschte Form geschnitten.

Die Konzentration des oder der pharmazeutischen Wirkstoffe in einem speziellen Pflaster gemäss der Erfindung hängt u.a. von den Freisetzungscharakteristika des Pflasters, der Grösse des Pflasters, der Potenz und den Charakteristika des oder der Wirkstoffe und der Dauer, während der der Wirkstoff zu verwenden ist, ab.

Im allgemeinen soll ein erfindungsgemässes Bukalpflaster für eine einmalige tägliche Verabreichung eines systemisch wirkenden Medikaments die ungefähr 0,1- bis 1-fache Menge der täglichen Dosis des Medikaments enthalten.

Die nachfolgenden Beispiele illustrieren die Erfindung; sie sollen jedoch diese in ihrem Umfang in keiner Weise einschränken.

Beispiel 1: Herstellung einer Polyvinylalkohol-Folie

a) Polyvinylalkohol (9,83 g, Mowiol 28-99 von Hoechst) wird in Wasser (88,5 g) gelöst und mit Glycerin (1,1 g) sowie rotem Eisenoxid (0,56 g) versetzt. Die so entstandene Masse wird auf eine glatte Oberfläche aus Glas 1 mm dick aufgetragen und getrocknet. Beim Abdampfen des Wassers entsteht eine ca. 0,1 mm dicke, feste Folie, die man durch Nachbehandlung beliebig formen und schneiden kann.

b) Man folgt der Arbeitsvorschrift des Beispiels 1a), verzichtet aber auf den Zusatz von rotem Eisenoxid.

Beispiel 2: Herstellung einer Klebemasse, die keinen Wirkstoff enthält

a) Titandioxid (0,94 g) und 0,33 g Methylcellulose (Methocel MC, Dow) werden in Ethanol (853,42 g) dispergiert. In diese Dispersion werden 37,72 g Polyacrylsäure (Carbopol 934P, Goodrich) zugefügt.

b) Ferner werden 3,77 g Polyoxyethylen(20)-Stearylether (BRIJ 78, Atlas Chemical Division) in 99,68 g Ethanol gelöst und mit 4,14 g Glycerin versetzt.

c) Dann werden die gemäss den Beispielen 2a) und 2b) erhaltenen Massen zu einem homogenen Gel vermengt.

d) Man folgt der Arbeitsvorschrift des Beispiels 2a), verzichtet aber auf den Zusatz von Titandioxid.

Beispiel 3: Herstellung einer Klebemasse enthaltend Heparin und Zinksulfat

Zu der Dispersion von Beispiel 2a), enthaltend Titandioxid, Methylcellulose und Polyacrylsäure in Ethanol, werden 3,12 g Zinksulfat-monohydrat und 0,95 g Heparin-natriumsalz gegeben. Dann folgt man der Arbeitsvorschrift von Beispiel 2b) und 2c).

Beispiel 4: Herstellung einer Klebemasse enthaltend Diclofenac-Natrium

Man folgt der Arbeitsvorschrift des Beispiels 3, verwendet jedoch anstelle von Heparin-Natriumsalz und Zinksulfat-monohydrat 2,13 g Diclofenac-Natrium.

Beispiel 5: Zusammensetzung des Systems

Auf eine Polyvinylalkohol-Folie, erhalten gemäss Beispiel 1, wird eine gleichmässig dicke Klebemasse, erhalten gemäss einem der Beispiele 2, 3 oder 4, aufgetragen und getrocknet. Dann wird das System in die gewünschte Form geschnitten, wobei der Wirkstoffgehalt z.B. gemäss Beispiel 3 0,15 mg Heparin/cm² und 0,5 mg Zinksulfat•H$_2$O/cm² beträgt.

Beispiel 6:
  Gemäss den Beispielen 1-5 werden folgende Systeme enthaltend einen pharmazeutischen Wirkstoff hergestellt:

| Wirkstoff | Wirkstoffgehalt $(\mu g/cm^2)$ |
|---|---|
| Progesteron | 450 |
| Nitroglycerin | 620 |
| Propranolol | 180 |
| Scopolamin-hydrobromid | 50 |
| Nikotin | 700 |
| Nupercain | 180 |
| Doxylamin-succinat | 100 |
| Lidocain | 160 |

## Patentansprüche

1. Pharmazeutisches, auf der Schleimhaut haftendes Pflaster bestehend aus zwei diskreten, gut zusammenhaftenden Schichten:
  (a) einer Abdeckfolie enthaltend als wesentliche Bestandteile:
  (1) kaltwasserunlöslichen Polyvinylalkohol, in dem höchstens 10 % der Hydroxygruppen als Acetatgruppen vorliegen: und
  (2) gegebenenfalls einen oder mehrere Weichmacher; und
  (b) einer Klebschicht enthaltend als wesentliche Bestandteile:
  (1) ein bei Körpertemperatur wasserunlösliches Assoziationspolymer, gebildet ($\alpha$) aus einer polymeren Carbonsäure, worin jeweils wenigstens 10 % der Monomereinheiten freie Carboxygruppen tragen, und ($\beta$) einem ethoxylierten, nichtionischen Tensid, das wenigstens 2 Ethylenoxid-Einheiten enthält und ein Molekulargewicht von maximal 4000 besitzt, in einem Gewichtsverhältnis von etwa 50:1 bis etwa 1:20;
  (2) gegebenenfalls einen oder mehrere pharmazeutische Wirkstoffe; und
  (3) gegebenenfalls einen oder mehrere die Absorption des (der) pharmazeutischen Wirkstoffe(s) fördernde Substanzen.
2. Pharmazeutisches Pflaster gemäss Anspruch 1, dadurch gekennzeichnet, dass die polymere Carbonsäure aus der Gruppe Polyacrylsäure, Polymaleinsäure, Polyacrylsäure-Copolymer, Polymaleinsäure-Copolymer und Alginsäure stammt und das ethyoxylierte, nichtionische Tensid ein Polyoxyethylenfettalkohol oder eine Polyoxyethylensorbitanfettsäure ist.
3. Pharmazeutisches Pflaster gemäss Anspruch 1, bestehend aus:
  (a) einer Abdeckfolie enthaltend als wesentliche Bestandteile:
  (1) kaltwasserunlöslichen Polyvinylalkohol, in dem höchstens 8 % der Hydroxygruppen als Acetatgruppen vorliegen; und
  (2) 1-30 Gew.-% eines oder mehrerer Weichmacher; und
  (b) einer Klebeschicht enthaltend als wesentliche Bestandteile:
  (1) ein Assoziationspolymer, gebildet ($\alpha$) aus einer polymeren Carbonsäure aus der Gruppe Polyacrylsäure und Alginsäure und ($\beta$) einem ethyoxylierten, nichtionischen Tensid aus der Gruppe Polyoxyethylenfettalkohole und Polyoxyethylensorbitanfettsäuren, in einem Gewichtsverhältnis von etwa 20:1 bis etwa 1:4;
  (2) gegebenenfalls einen oder mehrere pharmazeutische Wirkstoffe; und
  (3) gegebenenfalls einen oder mehrere die Absorption des (der) pharmazeutischen Wirkstoffe(s) fördernde Substanzen.
4. Pharmazeutisches Pflaster gemäss Anspruch 1, bestehend aus:
  (a) einer Abdeckfolie enthaltend als wesentliche Bestandteile:
  (1) kaltwasserunlöslichen Polyvinylalkohol, in dem höchstens 2 % der Hydroxygruppen als Acetatgruppen vorliegen; und

(2) 5-20 Gew.-% eines oder mehrerer Weichmacher; und

 (b) einer Klebeschicht enthaltend als wesentliche Bestandteile:

(1) ein Assoziationspolymer, gebildet ($\alpha$) aus Polyacrylsäure und ($\beta$) einem ethoxylierten, nichtionischen Tensid aus der Gruppe polyoxyethylierter Stearylalkohol, polyoxyethylierter Palmitylalkohol, Polyoxyethylensorbitanstearinsäure und Polyoxethylensorbintanpalmitinsäure, in einem Gewichtsverhältnis von etwa 15:1 bis etwa 5:1;

(2) gegebenenfalls einen oder mehrere pharmazeutische Wirkstoffe; und

(3) gegebenenfalls einen oder mehrere die Absorption des (der) pharmazeutischen Wirkstoffe(s) fördernde Substanzen.

5. Pharmazeutisches Pflaster gemäss Anspruch 4, dadurch gekennzeichnet, dass der Weichmacher in der Abdeckfolie Glycerin und das ethoxylierte, nichtionische Tensid in der Klebeschicht polyoxyethylierter Stearylalkohol ist.

6. Pharmazeutisches Pflaster gemäss einem der Ansprüche 1-5, dadurch gekennzeichnet, dass die Abdeckfolie anders gefärbt ist als die Klebeschicht.

7. Pharmazeutisches Pflaster gemäss einem der Ansprüche 1-6, dadurch gekennzeichnet, dass es keinen pharmazeutischen Wirkstoff enthält.

8. Pharmazeutisches Pflaster gemäss einem der Ansprüche 1-6, dadurch gekennzeichnet, dass es einen oder mehrere pharmazeutische Wirkstoffe enthält.

9. Pharmazeutisches Pflaster gemäss Anspruch 8, dadurch gekennzeichnet, dass es mindestens einen Wirkstoff aus der Gruppe Analgetika und Antiinflammatorika enthält.

10. Pharmazeutisches Pflaster gemäss Anspruch 8, dadurch gekennzeichnet, dass es mindestens ein Anästhetikum oder Lokalanästhetikum enthält.

11. Pharmazeutisches Pflaster gemäss Anspruch 8, dadurch gekennzeichnet, dass es mindestens ein antivirales Mittel enthält.

12. Pharmazeutisches Pflaster gemäss Anspruch 11, dadurch gekennzeichnet, dass es als antivirales Mittel ein Gemisch aus Heparin und Zinksalzen enthält.

13. Pharmazeutisches Pflaster gemäss Anspruch 8, dadurch gekennzeichnet, dass es mindestens einen Peptidwirkstoff enthält.

14. Pharmazeutisches Pflaster gemäss Anspruch 8, dadurch gekennzeichnet, dass es mindestens ein Hormon enthält.

15. Pharmazeutisches Pflaster gemäss Anspruch 8, dadurch gekennzeichnet, dass es mindestens ein antianginöses Präparat enthält.

16. Pharmazeutisches Pflaster gemäss Anspruch 8, dadurch gekennzeichnet, dass es mindestens einen $\beta$-Blocker enthält.

17. Pharmazeutisches Pflaster gemäss Anspruch 8, dadurch gekennzeichnet, dass es mindestens ein Entwöhnungsmittel enthält.

18. Pharmazeutisches Pflaster gemäss Anspruch 8, dadurch gekennzeichnet, dass es mindestens ein Mittel gegen die Reisekrankheit enthält.

19. Pharmazeutisches Pflaster gemäss einem der Ansprüche 8-18, dadurch gekennzeichnet, dass es zusätzlich in der Klebeschicht mindestens eine penetrationsfördernde Substanz enthält.

20. Pharmazeutisches Pflaster gemäss einem der Ansprüche 1-19 zur Verwendung als Bukalpflaster.

21. Verfahren zur Herstellung eines pharmazeutischen Pflasters gemäss einem der Ansprüche 1-19, dadurch gekennzeichnet, dass man die Abdeckfolie mit einer Klebemasse bestehend aus den Bestandteilen der Klebeschicht und einem Lösungsmittel gleichmässig beschichtet, das Lösungsmittel verdampft und das Produkt in die gewünschte Form bringt.

Patentansprüche für die Folgenden Vertragsstaaten: ES, GR

1. Verfahren zur Herstellung eines pharmazeutischen, auf der Schleimhaut haftenden Pflasters bestehend aus zwei diskreten, gut zusammenhaftenden Schichten:

(a) einer Abdeckfolie enthaltend als wesentliche Bestandteile:

(1) kaltwasserunlöslichen Polyvinylalkohol, in dem höchstens 10 % der Hydroxygruppen als Acetatgruppen vorliegen; und

(2) gegebenenfalls einen oder mehrere Weichmacher; und

(b) einer Klebeschicht enthaltend als wesentliche Bestandteile:

(1) ein bei Körpertemperatur wasserunlösliches Assoziationspolymer, gebildet ($\alpha$) aus einer polymeren Carbonsäure, worin jeweils wenigstens 10 % der Monomereinheiten freie Carboxygruppen tragen, und ($\beta$) einem ethoxylierten, nichtionsichen Tensid, das wenigstens 2 Ethylenoxid-Einheiten enthält und ein Molekulargewicht von maximal 4000 besitzt, in einem Gewichtsverhältnis von etwa 50:1 bis etwa 1:20;

(2) gegenbenenfalls einem oder mehrere pharmazeutische Wirkstoffe; und

(3) gegebenenfalls einen oder mehrere die Absorption des (der) pharmazeutischen Wirkstoffe(s) fördernde Substanzen,

dadurch gekennzeichnet, dass man die Abdeckfolie mit einer Klebemasse bestehend aus den Bestandteilen der Klebeschicht und einem Lösungsmittel gleichmässig beschichtet, das Lösungsmittel verdampft und das Produkt in die gewünschte Form bringt.

2. Verfahren gemäss Anspruch 1, dadurch gekennzeichnet, dass ein pharmazeutisches Pflaster, worin die polymere Carbonsäure aus der Gruppe Polyacrylsäure, Polymaleinsäure, Polyacrylsäure-Copolymer, Polymaleinsäure-Copolymer und Alginsäure stammt und das ethoxylierte, nichtionische Tensid ein Polyoxyethylenfettalkohol oder eine Polyoxyethylensorbitanfettsäure ist, hergestellt wird.

3. Verfahren gemäss Anspruch 1, dadurch gekennzeichnet, dass ein pharmazeutisches Pflaster bestehend aus:

(a) einer Abdeckfolie enthaltend als wesentliche Bestandteile:

(1) kaltwasserunlöslichen Polyvinylalkohol, in dem höchstens 8 % der Hydroxygruppen als Acetatgruppen vorliegen; und

(2) 1-30 Gew.-% eines oder mehrerer Weichmacher; und

(b) einer Klebeschicht enthaltend als wesentliche Bestandteile:

(1) ein Assoziationspolymer, gebildet ($\alpha$) aus einer polymeren Carbonsäure aus der Gruppe Polyacrylsäure und Alginsäure und ($\beta$) einem ethoxylierten, nichtionischen Tensid aus der Gruppe Polyoxyethylenfettalkohole und Polyoxyethylensorbitanfettsäuren, in einem Gewichtsverhältnis von etwa 20:1 bis etwa 1:4;

(2) gegebenenfalls einen oder mehrere pharmazeutische Wirkstoffe; und

(3) gegebenenfalls einen oder mehrere die Absorption des (der) pharmazeutischen Wirkstoffe(s) fördernde Substanzen, hergestellt wird.

4. Verfahren gemäss Anspruch 1, dadurch gekennzeichnet, dass ein pharmazeutisches Pflaster bestehend aus:

(a) einer Abdeckfolie enthaltend als wesentliche Bestandteile:

(1) kaltwasserunlöslichen Polyvinylalkohol, in dem höchstens 2 % der Hydroxygruppen als Acetatgruppen vorliegen; und

(2) 5-20 Gew.-% eines oder mehrerer Weichmacher; und

(b) einer Klebeschicht enthaltend als wesentliche Bestandteile:

(1) ein Assoziationspolymer, gebildet ($\alpha$) aus Polyacrylsäure und ($\beta$) einem ethoxylierten, nichtionsichen Tensid aus der Gruppe polyoxyethylierter Stearylalkohol, polyoxyethylierter Palmitylalkohol, Polyoxyethylensorbitanstearinsäure und Polyoxyethylensorbitanpalmitinsäure, in einem Gewichtsverhältnis von etwa 15:1 bis etwa 5:1;

(2) gegebenenfalls einen oder mehrere pharmazeutische Wirkstoffe; und

(3) gegebenenfalls einen oder mehrere die Absorption des (der) pharmazeutischen Wirkstoffe(s) fördernde Substanzen, hergestellt wird.

5. Verfahren gemäss Anspruch 4, dadurch gekennzeichnet, dass ein pharmazeutisches Pflaster, worin der Weichmacher in der Abdeckfolie Glycerin und das ethyoxylierte, nichtionische Tensid in der Klebeschicht polyoxyethylierter Stearylalkohol ist, hergestellt wird.

6. Verfahren gemäss einem der Ansprüche 1-5, dadurch gekennzeichnet, dass ein pharmazeutisches Pflaster, worin die Abdeckfolie anders gefärbt ist als die Klebeschicht, hergestellt wird.

7. Verfahrn gemäss einem der Ansprüche 1-6, dadurch gekennzeichnet, dass ein pharmazeutisches Pflaster, welches keinen pharmazeutischen Wirkstoff enthält, hergestellt wird.

8. Verfahren gemäss einem der Ansprüche 1-6, dadurch gekennzeichnet, dass ein pharmazeutisches Pflaster, welches einen oder mehrere pharmazeutische Wirkstoffe enthält, hergestellt wird.

9. Verfahren gemäss Anspruch 8, dadurch gekennzeichnet, dass ein pharmazeutisches Pflaster, welches mindestens einen Wirkstoff aus der Gruppe Analgetika und Antiinflammatorika enthält, hergestellt wird.

10 Verfahren gemäss Anspruch 8, dadurch gekennzeichnet, dass ein pharmazeutisches Pflaster, welches mindestens ein Anästhetikum oder Lokalanästhetikum enthält, hergestellt wird.

11. Verfahren gemäss Anspruch 8, dadurch gekennzeichnet, dass ein pharmazeutisches Pflaster, welches mindestens ein antivirales Mittel enthält, hergestellt wird.

12. Verfahren gemäss Anspruch 11, dadurch gekennzeichnet, dass ein pharmazeutisches Pflaster, welches ein Gemisch aus Heparin und Zinksalzen als antivirales Mittel enthält, hergestellt wird.

13. Vefahren gemäss Anspruch 8, dadurch gekennzeichnet, dass ein pharmazeutisches Pflaster, welches mindestens einen Peptidwirkstoff enthält, hergestellt wird.

14. Verfahren gemäss Anspruch 8, dadurch gekennzeichnet, dass ein pharmazeutisches Pflaster, welches mindestens ein Hormon enthält, hergestellt wird.

15. Verfahren gemäss Anspruch 8, dadurch gekennzeichnet, dass ein pharmazeutisches Pflaster, welches mindestens ein antianginöses Präparat enthält, hergestellt wird.

16. Verfahren gemäss Anspruch 8, dadurch gekennzeichnet, dass ein pharmazeutisches Pflaster, welches mindestens einen $\beta$-Blocker enthält, hergestellt wird.

17. Verfahren gemäss Anspruch 8, dadurch gekennzeichnet, dass ein pharmazeutisches Pflaster, welches mindestens ein Entwöhnungsmittel enthält, hergestellt wird.

18. Verfahren gemäss Anspruch 8, dadurch gekennzeichnet, dass ein pharmazeutisches Pflaster, welches mindestens ein Mittel gegen die Reisekrankheit enthält, hergestellt wird.

19. Verfahren gemäss einem der Ansprüche 8-18, dadurch gekennzeichnet, dass ein pharmazeutisches Pflaster, welches zusätzlich in der Klebeschicht mindestens eine penetrationsfördernde Substanz enthält, hergestellt wird.

20. Verfahren gemäss einem der Ansprüche 1-19, dadurch gekennzeichnet, dass ein Bukalpflaster hergestellt wird.